# DEMANDE DE BREVET EUROPEEN

(11) **EP 1 892 004 A2**
(43) Date de publication de la demande: **27.02.2008**
(21) Numéro de dépôt: 07120297.2
(22) Date de dépôt: 09.06.1999
(51) Int. Cl.: A61M 5/32

(54) **Ensemble de sécurité pour une seringue pré-remplie d'injection de liquide, notamment d'un médicament**

(30) Priorité: 08.12.1998 FR 9815628
(62) Demande divisionnaire de: 03290416.1
(71) Demandeur: Rexam Pharma La Verpillière, 38290 La Verpilliere Cedex (FR)
(72) Inventeur: Denis, Roger M., (FR)
(74) Mandataire: Thiollier, Clémence-Olivia Laure Marie

(57) **Abrégé**

L'invention concerne un ensemble de sécurité (E) pour une seringue (S), comprenant :
- un fourreau tubulaire (1) dans lequel le corps de seringue (K) est destiné à être logé déplaçable axialement,
- des moyens de rappel élastique (R),
- des moyens de blocage (5) destinés à immobiliser le corps (K) par rapport au fourreau (1), ces moyens de blocage étant libérables,
- un capuchon de révolution (8), monté coulissant axialement par rapport au fourreau (1),
- des ergots intérieurs (10) pour l'encliquetage de la collerette (CL) du corps de seringue (K) sur le capuchon (8), et
- deux épaulements (7, 11) complémentaires formant butées, prévus respectivement sur le capuchon (8) et le fourreau (1) pour limiter la course du capuchon (8) après libération des moyens de blocage (5).

## Description

La présente invention concerne un ensemble de sécurité pour une seringue pré-remplie d'injection de liquide, notamment d'un médicament.

On connaît déjà dans l'état de la technique un ensemble de sécurité pour une seringue d'injection de liquide pré-remplie comportant un corps tubulaire, formant un réservoir pour le liquide, portant une aiguille d'injection du liquide, et un poussoir monté déplaçable dans le corps entre des positions d'attente et de fin d'injection.

Habituellement, le poussoir de la seringue comporte une extrémité de manoeuvre externe au corps de la seringue et une extrémité interne au corps de cette seringue portant un piston. L'extrémité de manoeuvre du poussoir est encore appelée tête de poussoir.

Certains produits médicaux tels que les vaccins, les héparines et leurs dérivés et autres médicaments à dosages précis, ne peuvent être stockés dans les conteneurs ou seringues en plastique par suite de taux de reprise d'humidité qui risquent d'en compromettre la composition.

Conscients de ces impératifs, les laboratoires sont astreints à n'utiliser que des conteneurs en verre, qui ne présentent aucune porosité. Compte tenu des maladies infectieuses à virus jusqu'alors sans antidote, une sécurité est actuellement recherchée, permettant une protection de tous les personnels soignants contre les risques de contaminations éventuelles lors d'accidents professionnels dus à des piqûres. Cette protection doit être proche du taux de 100% réclamé par la profession.

Le problème est donc d'adapter un mécanisme de sécurité sur des seringues de préférence en verre, notamment de 0,1 à 2 ml de capacité, tout type de mécanisme interne à ces seringues étant exclu.

D'autre part, l'impératif de stérilité interdit toute manipulation interne de la seringue après remplissage.

Quelques solutions ont été présentées aux laboratoires et écartées pour différents motifs : augmentation du volume, modification des lignes de remplissage, opérations complexes pour le médecin ou les personnels devant mettre ces produits en oeuvre, pseudo-protection plus nuisible qu'utile.

On notera les libertés de langage critiquables prises par de nombreux inventeurs dans les appellations telles que « seringues à aiguille auto-rétractable », qui en fait ne sont, très souvent, que des seringues auto-bloquantes, ou à éléments destructibles, ou à capuchon ou tube coulissant occultant l'aiguille après usage.

Dans la pratique une infirmière tient une seringue entre l'index et le majeur et appuie avec le pouce sur la tête du poussoir. De ce fait, la seringue étant maintenue, la rétraction de l'aiguille ne peut s'opérer que dans un poussoir ou piston creux, le corps de la seringue demeurant immobile.

Dans les autres cas, un fourreau coulissant longitudinalement sur l'extérieur de la seringue vient « coiffer » l'aiguille ce qui présente en fait un « escamotage de l'aiguille » par avancement du fourreau soit manuel, soit automatique.

Cet agencement connu est à la base de nombreux brevets, et, a été réfuté par la profession médicale par suite du risque d'arrachage ou d'incision résultant du choc du fourreau venant en biais percuter le patient sous la force d'un ressort de rappel du fourreau en position d'escamotage de l'aiguille.

L'invention a pour but de remédier aux inconvénients exposés ci-dessus, tout en répondant aux critères suivants: faible encombrement, adaptation en fin de chaîne avant emballage, sécurité à déclenchement unimanuel, automatique, à commande indépendante de la volonté humaine, protection la plus voisine des 100% souhaités.

A cet effet, l'invention a pour objet un ensemble de sécurité pour une seringue d'injection de liquide pré-remplie comportant un corps tubulaire, formant un réservoir pour le liquide, portant une aiguille d'injection du liquide, et un poussoir monté déplaçable dans le corps entre des positions d'attente et de fin d'injection, comprenant :
- un fourreau tubulaire dans lequel le corps de la seringue est destiné à être logé déplaçable axialement entre une position active, dans laquelle l'aiguille fait saillie à travers une extrémité distale du fourreau, et une position de protection, dans laquelle l'aiguille est escamotée dans le fourreau,
- des moyens de rappel élastique du corps vers sa position de protection et
- des moyens de blocage destinés à immobiliser le corps par rapport au fourreau en position active en s'opposant à la force élastique des moyens de rappel, ces moyens de blocage étant libérables par des moyens activés lorsque le poussoir est en position de fin d'injection, les moyens de blocage comprenant des moyens escamotables formés par deux pattes diamétralement opposées formées dans la paroi du fourreau et terminées par des ergots escamotables,
caractérisé en ce qu'il comporte :
- un capuchon de révolution, de préférence en matière plastique, monté coulissant axialement par rapport au fourreau entre deux positions et traversé par le poussoir qui s'étend à travers un orifice ménagé dans l'extrémité proximale de ce capuchon,
- des ergots intérieurs pour l'encliquetage de la collerette du corps de seringue sur le capuchon, de sorte que du fait de l'escamotage des ergots escamotables, le capuchon se déplace également sous l'effet de la force de rappel des moyens de rappel, et
- deux épaulements complémentaires formant butées, prévus respectivement sur le capuchon et le fourreau pour limiter la course du capuchon après libération des moyens de blocage du corps, en s'opposant à la force élastique de rappel des moyens de rappel.

Suivant un mode de réalisation, les moyens de rappel comprennent un ressort de poussée destiné à venir en appui sur un épaulement interne d'appui ménagé dans le fourreau, lequel comporte par exemple un logement interne de révolution dans lequel est disposé le ressort de poussée.

Ce logement interne est, par exemple, délimité par l'épaulement interne d'appui du ressort de poussée.

L'ensemble de sécurité peut comporter des moyens pour bloquer la collerette du corps de seringue après détente des moyens de rappel.

Suivant un mode de réalisation, le fourreau et le capuchon comportent des moyens complémentaires d'immobilisation en rotation de l'un par rapport à l'autre.

Par ailleurs, le fourreau peut porter des moyens de préhension externes destinés à être saisis par les doigts d'un utilisateur pour injecter le liquide par rapprochement axial d'une extrémité de manoeuvre du poussoir et des moyens de préhension.

L'invention sera mieux comprise à la lecture de la description qui va suivre donnée uniquement à titre d'exemple et faite en se référant aux dessins dans lesquels :
- les figures 1 à 3 sont des vues en coupe axiale d'un ensemble de sécurité pour une seringue selon un premier mode de réalisation de l'invention dans trois configurations d'utilisation respectivement ;
- les figures 4 et 5 sont des vues d'un ensemble de sécurité pour seringue selon un second mode de réalisation de l'invention en coupe axiale suivant deux plans perpendiculaires entre eux, le poussoir de la seringue étant dans sa position d'attente ;
- la figure 6 est une vue similaire à la figure 5, dans laquelle le poussoir de la seringue est en position de fin d'injection ;
- la figure 7 est une vue similaire à la figure 4, montrant la position du poussoir de la seringue après libération des moyens de blocage du corps de la seringue ;
- les figures 8 à 10 sont des vues en coupe axiale d'un ensemble de sécurité pour une seringue selon un troisième mode de réalisation de l'invention, dans trois configurations d'utilisation respectivement ;
- la figure 11 est une vue en coupe axiale du fourreau de protection de l'ensemble d'injection représenté sur les figures 8 à 10 ;
- les figures 12 et 13 sont des vues du capuchon de l'ensemble d'injection représenté sur les figures 8 à 10, en coupe axiale suivant deux plans perpendiculaires entre eux.

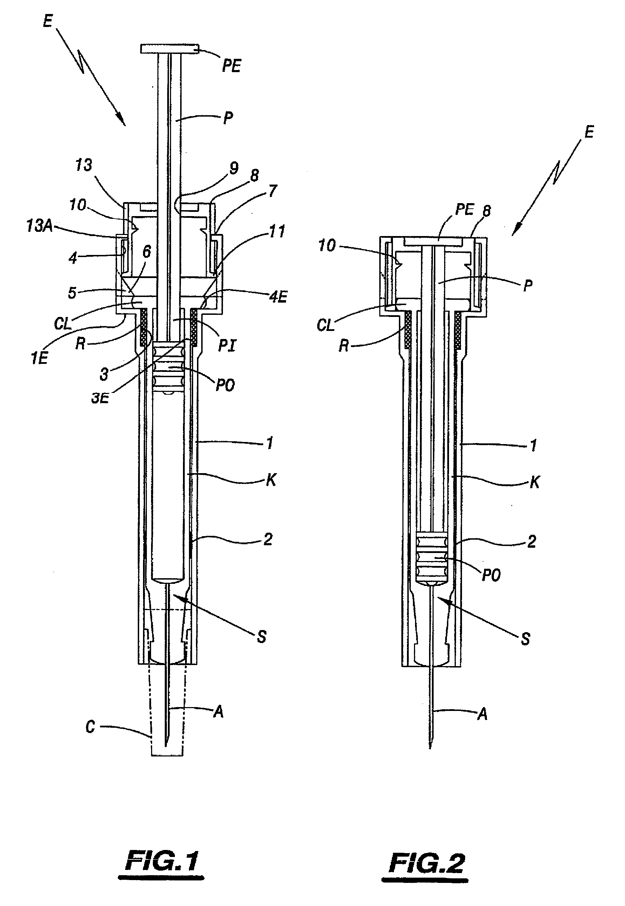

On a représenté sur les figures 1 à 3 un ensemble de sécurité pour une seringue classique S d'injection de liquide, notamment un liquide médical, selon un premier mode de réalisation de l'invention. Cet ensemble de sécurité est désigné par la référence générale E.

La seringue S est pré-remplie, prête à l'emploi, et comporte un corps tubulaire K, formant un réservoir pour le liquide, portant une aiguille A d'injection du liquide, et un poussoir P monté déplaçable dans le corps K entre une position d'attente, telle que représentée sur la figure 1, et une position de fin d'injection de liquide, telle que représentée sur la figure 2.

Le poussoir P comporte une première extrémité PE, externe au corps K, et une seconde extrémité Pl, interne au corps K, portant un piston classique PO. L'extrémité externe PE forme une extrémité de manoeuvre du poussoir.

L'ensemble E comporte un fourreau de sécurité tubulaire dans lequel est logé la seringue, notamment le corps K. Le fourreau, de révolution, est constitué par exemple, par un tube cylindrique 1 en matière plastique ou en tout autre matériau ayant une transparence suffisante pour permettre la visualisation du liquide contenu dans le corps K de la seringue ainsi que d'une étiquette éventuelle d'identification du médicament. On notera que le tube cylindrique 1 est étagé.

Dans ce qui suit un élément sera qualifié de proximal ou distal selon qu'il est proche ou éloigné axialement de l'extrémité externe PE du poussoir.

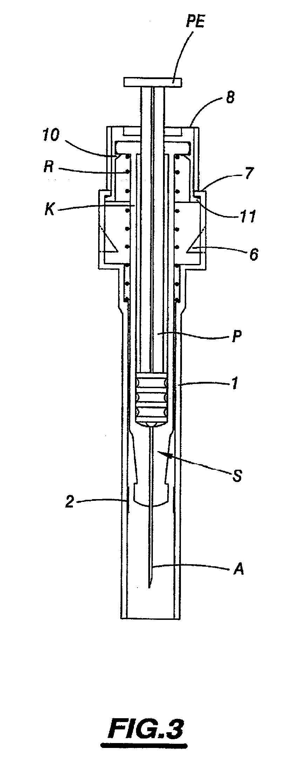

Le corps K est déplaçable axialement dans le tube 1 entre une position active dans laquelle l'aiguille A fait saillie à travers une extrémité distale (supérieure) du tube 1, et une position de protection dans laquelle l'aiguille A est escamotée dans le tube 1. La position active est représentée sur les figures 1 et 2. La position de protection est représentée sur la figure 3.

On notera que le corps K de la seringue comporte une extrémité distale portant l'aiguille A et une extrémité proximale munie d'une collerette CL.

La surface interne du tube 1 comporte trois guides 2 en relief disposés dans le sens longitudinal de ce tube 1, espacés entre eux de 120°, destinés à centrer le corps K de la seringue, réduire le jeu et améliorer le coulissement du corps K dans le tube 1, tout en permettant le passage d'une cape en caoutchouc classique C (représentée an traits mixtes sur la figure 1) protégeant l'aiguille A avant utilisation de la seringue.

L'extrémité proximale du tube 1 porte, de bas en haut, un premier logement interne de révolution 3 prolongé par un second logement interne de révolution 4 de diamètre supérieur à celui du premier logement interne.

Le corps K est immobilisé par rapport au tube 1 à l'aide de moyens de blocage comprenant, d'une part, un siège 4E d'appui de la collerette CL formé par un épaulement interne délimitant le second logement 4, et d'autre part, des moyens escamotables de serrage de la collerette CL contre ce siège 4E, portés par l'extrémité proximale du tube 1.

Dans l'exemple illustré sur les figures 1 à 3, les moyens de serrage escamotables sont formés par deux pattes 5, sensiblement diamétralement opposées, partiellement découpées dans la paroi du tube 1 délimitant le second logement 4. Les pattes 5 sont terminées par des ergots escamotables 6 destinés à maintenir la collerette CL du corps de la seringue en appui contre le siège 4E. On notera que les pattes 5 sont pliées de façon à s'étendre sensiblement dans un plan longitudinal diamétral du tube. En variante, les moyens de serrage escamotables pourraient ne comporter qu'une seule patte 5.

Le corps K de la seringue est rappelé élastiquement vers sa position de protection par un ressort de poussée R disposé dans le premier logement 3. Ce ressort R est en appui, d'une part, sur un épaulement interne 3E, délimitant le premier logement interne 3, et d'autre part, sur la collerette CL du corps K de la seringue. L'épaulement interne d'appui 3E est disposé entre le siège 4E d'appui de la collerette CL et l'extrémité distale du tube 1.

Sur la figure 1, qui représente l'ensemble de sécurité et la seringue prêts à être utilisés, le ressort R est comprimé. Ce ressort reste dans cet état jusqu'à la fin de l'injection du liquide.

Les ergots 6 permettent donc d'immobiliser le corps K par rapport au tube 1 en position active en s'opposant à la force élastique de rappel du ressort R.

On notera que l'extrémité proximale du second logement interne 4 est délimitée par un anneau intérieur 7 venu de moulage avec le tube 1.

Les moyens de blocage du corps K de la seringue par rapport au tube 1 sont libérables à l'aide de moyens activés lorsque le poussoir P est en position de fin d'injection.

Dans l'exemple illustré sur les figures 1 à 3, les moyens de libération des moyens de blocage comprennent un capuchon de révolution 8, de préférence en matière plastique, monté coulissant axialement dans le second logement 4 du tube 1, entre une position haute d'attente, telle que représentée sur la figure 1, et une position d'escamotage des ergots 6, telle que représentée sur la figure 2. Le capuchon 8 est traversé par le poussoir P qui s'étend à travers un trou 9 ménagé dans l'extrémité proximale de ce capuchon 8.

Le capuchon 8 comporte également des ergots intérieurs 10 espacés entre eux de 90°, ainsi qu'un anneau extérieur 11 venu de moulage avec l'extrémité distale de ce capuchon.

L'anneau intérieur 7 du second logement 4 et l'anneau extérieur 11 du capuchon .8 délimitent des épaulements annulaires complémentaires, formant butées, limitant la course de ce capuchon 8, après libération des moyens de blocage du corps K, en s'opposant à la force élastique de rappel du ressort R (voir figure 3). Les anneaux complémentaires 7, 11 font donc office de limiteur de retour du capuchon 8.

De préférence, selon le type de seringue notamment, le tube 1 et le capuchon 8 comportent des moyens complémentaires d'immobilisation en rotation de l'un par rapport à l'autre. Ces moyens complémentaires d'immobilisation en rotation comprennent, par exemple, au moins une rainure longitudinale 13, ménagée dans le capuchon 8, coopérant par emboîtement avec un doigt correspondant 13A prolongeant radialement l'anneau intérieur 7 du second logement 4. De préférence, le capuchon comporte deux rainures 13 diamétralement opposées comme cela est illustré sur les figures 1 à 3.

La mise en place de la seringue dans l'ensemble de sécurité E peut être réalisée de la façon suivante.

Initialement, le corps K de la seringue est rempli de liquide à injecter. Ce liquide est retenu dans le corps K par le piston PO. Le poussoir P est séparé de ce piston.

On place le corps K de la seringue pré-remplie dans le tube 1 en encliquetant la collerette CL sous les ergots 6 après avoir comprimé le ressort R dans le premier logement 3.

Ensuite, le capuchon 8 est engagé dans le second logement 4 en étant par exemple enfoncé dans ce second logement 4 jusqu'à ce que l'anneau extérieur 11 arrive au contact des ergots 6, comme cela est représenté sur la figure 1.

Enfin, le poussoir P de la seringue est relié au piston PO en introduisant l'extrémité distale de ce poussoir dans le capuchon 8 et le tube 1, à travers l'orifice 9 de ce capuchon 8.

L'ensemble de sécurité E est alors prêt à l'emploi dans la configuration illustrée sur la figure 1, l'aiguille A de la seringue faisant saillie à travers l'extrémité distale du tube 1.

Afin d'obtenir un effet d'aiguille et de seringue auto-rétractable, il est impératif que la préhension de l'ensemble de sécurité E soit réalisée au niveau du tube de protection 1 ce qui permet à la seringue munie de son aiguille de se rétracter librement, parfaitement en ligne, et dès la fin de l'injection, c'est-à-dire sans risque pour le patient. C'est ainsi que l'on obtient un ensemble E correctement désigné comme étant du type à aiguille auto rétractable.

La préhension au niveau du tube 1 est réalisée à l'aide de moyens de préhension externes, portés par le tube 1, destinés à être saisis par les doigts d'un utilisateur pour injecter le liquide par rapprochement axial de l'extrémité externe PE du poussoir et des moyens de préhension.

Dans l'exemple illustré sur les figures 1 à 3, les moyens de préhension du tube 1 comprennent un épaulement 1 E ménagé sur la surface externe du tube 1.

Pour réaliser l'injection du liquide, l'utilisateur saisit l'ensemble E entre l'index et le majeur. Le pouce exerce une pression de la seringue sur l'extrémité externe PE du poussoir. L'index et le majeur retiennent le tube 1 en saisissant l'épaulement de préhension 1 E.

L'utilisateur enfonce le poussoir PE dans le corps de la seringue en rapprochant axialement l'extrémité PE du poussoir de l'épaulement de préhension 1E, ceci jusqu'à atteindre la position de fin d'injection représentée sur la figure 2.

La course de déplacement du poussoir P depuis sa position d'attente jusqu'à sa position de fin d'injection comporte une course finale d'assujettissement de ce poussoir P au capuchon B précédée d'une course morte initiale du poussoir P par rapport au capuchon 8 au cours de laquelle ce capuchon 8 reste immobile. Au cours de la course d'assujettissement du poussoir P au capuchon 8, l'extrémité externe PE du poussoir, formant tête d'assujettissement, coopère avec un lamage délimitant le bord de l'orifice 9 du capuchon.

Du fait de l'entraînement du capuchon 8 par le poussoir P, l'anneau extérieur 11 de ce capuchon coopère avec les ergots 6 de façon à les escamoter, comme cela est représenté sur la figure 2. Sur cette figure 2, on voit que le corps K de la seringue est vidé de son contenu, le liquide ayant été injecté dans le corps d'un patient par l'utilisateur. Le poussoir P est en fin de course.

Du fait de l'escamotage des ergots 6, et du relâchement de la pression exercée par le pouce de l'utilisateur sur l'extrémité externe PE du poussoir, le ressort R se détend et pousse la seringue vide vers le haut (en considérant les figures) de façon à encliqueter la collerette CL du corps entre le bord de l'orifice 9 du capuchon et les ergots 10 comme cela est représenté sur la figure 3. Le capuchon 8 se déplace également sous l'effet de la force de rappel du ressort R vers la position représentée sur la figure 3 dans laquelle les anneaux 7, 11 coopèrent entre eux pour limiter la course du capuchon 8 et donc la course de rétraction de la seringue. Dans la configuration illustrée sur la figure 3, l'aiguille A est escamotée dans le tube 1 en réalisant ainsi une sécurité à 100%.

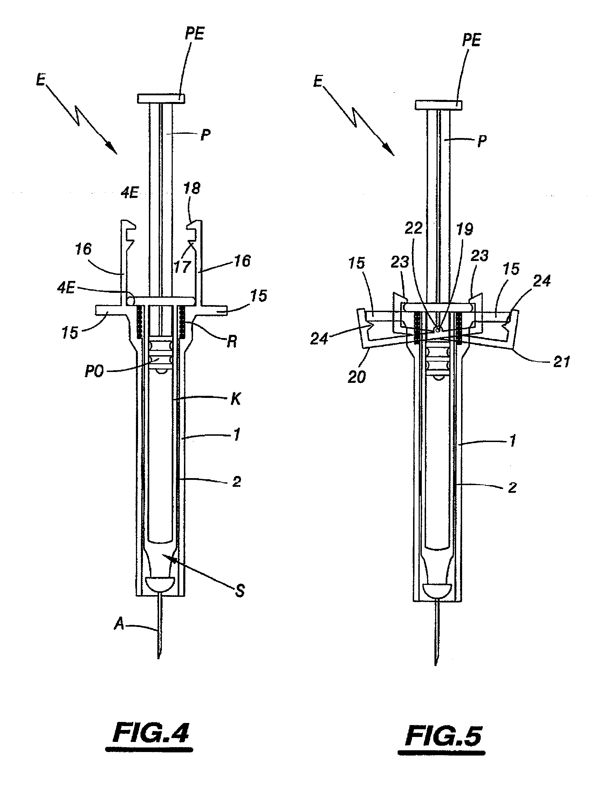

Sur les figures 4 à 7, on a représenté un ensemble de sécurité E selon un second mode de réalisation de l'invention. Sur ces figures, les éléments analogues à ceux des figures précédentes, sont désignés par des références identiques.

Dans ce cas, le tube 1 ne comporte pas le second logement interne du premier mode de réalisation. Par ailleurs, les moyens de préhension du tube 1 sont constitués par deux ailettes ou oreilles 15, sensiblement transversales à l'axe du tube, diamétralement opposées, prolongées dans le sens longitudinal (parallèlement à l'axe du tube 1) par deux pattes 16 terminées par des ergots inversés 17, 18. Les oreilles 15 sont, de préférence, venues de moulage avec le tube 1.

A 90° par rapport aux oreilles de préhension 15, également venus de moulage avec le tube 1, deux pivots 19 font saillie sur la surface externe de l'extrémité proximale du tube 1. Les pivots 19 matérialisent un axe géométrique sensiblement transversal au tube 1.

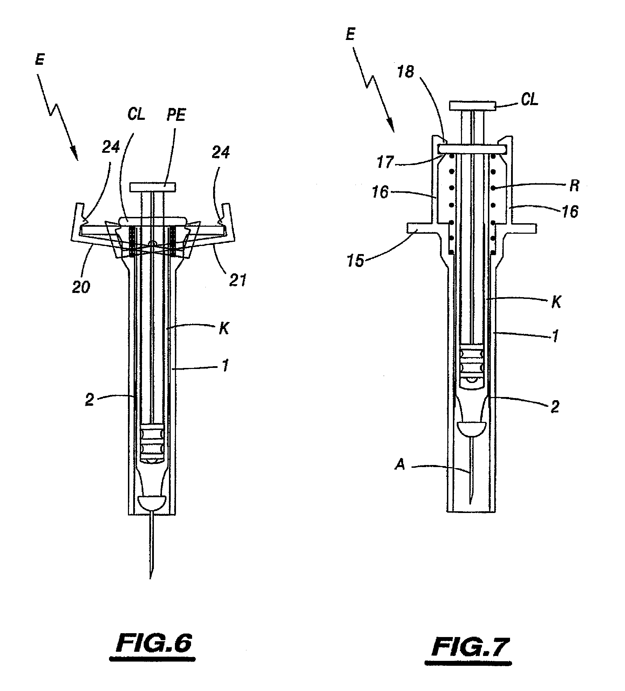

Les deux pivots 19 portent deux bras 20, 21 identiques, en forme générale de U très ouvert. Ces bras 20, 21 sont inversés par leur positionnement à 180°. Chaque bras 20, 21 comporte un trou de pivotement 22 pour emboîtage sur le pivot 19 correspondant. Chaque bras 20, 21 comporte des parties courte et longue séparées par le pivot 22 correspondant. La partie courte est terminée par une extrémité coudée sensiblement à angle droit, portant un ergot 23. La partie longue est également terminée par une extrémité coudée sensiblement à angle droit, comportant un bossage intérieur 24 (voir figures 5 et 6).

Les deux bras 20, 21, articulés autour d'un axe géométrique commun, forment une pince de serrage escamotable maintenant la collerette CL du corps de la seringue en appui contre le siège 4E délimité par le bord de l'extrémité proximale du tube 1.

En effet, les extrémités coudées des parties courtes et longues des bras 20, 21 forment, respectivement, des extrémités de serrage de la collerette CL contre le siège 4E et des extrémités de manoeuvre de ces bras 20, 21.

Lors de la mise en place de la seringue dans le tube 1, les ergots 23 viennent retenir la collerette CL de la seringue dans le tube 1 tout en comprimant le ressort R dans le logement 3.

Les bras 20, 21 sont placés dans une position de serrage forcé de la collerette CL du fait du positionnement de leur bossage 24 en-dessous des oreilles de préhension 15. Ce positionnement est symétrique du fait de la disposition des bras 20, 21 à 180° l'un de l'autre (voir figure 5).

Pour injecter le liquide dans le corps d'un patient, l'utilisateur applique deux doigts (en général l'index et le majeur) à la fois sur les oreilles 15 et les bras 20, 21.

Au cours de cette injection, les extrémités de manoeuvre des bras 20, 21, coopèrent avec les oreilles 15 de façon à être déplacées, lorsque le poussoir atteint sa position de fin d'injection, de la position de serrage forcée de la collerette CL représentée sur la figure 5, jusqu'à une position de libération forcée de cette collerette CL, représentée sur la figure 6. Le passage de la position de serrage forcé à la position de libération forcée de la collerette se fait par franchissement d'un point dur et déformation élastique des bras 20, 21. En effet, à la fin de l'injection, l'utilisateur appuie énergiquement sur l'extrémité externe PE du poussoir afin d'expulser l'intégralité du liquide contenu dans la seringue. Par ce geste, les bossages 24 échappent aux oreilles 15, ce qui écarte les ergots 23 de la collerette CL et libère le ressort R.

En se détendant, le ressort R pousse la seringue entre les pattes 16. La collerette CL du corps de la seringue coopère alors par encliquetage avec les deux pattes 16 prolongeant l'extrémité proximale du tube 1. Le blocage de la collerette CL, entre les ergots 17, 18, limite la course du corps K de la seringue après détente du ressort R comme cela est représenté sur la figure 7. Sur cette figure 7, l'aiguille de la seringue est escamotée dans le tube 1 après avoir été automatiquement rétractée de façon à être protégée dans le tube 1 de tout contact possible.

On notera que les seringues pré-remplies de vaccin doivent être conservées et transportées à basse température. Pour des raisons économiques et pratiques, on souhaite réduire autant que possible les dimensions des moyens de protection de l'aiguille, toute augmentation du volume de ces moyens de protection ayant une incidence sur le stockage et le transport des ensembles d'injection. De plus, l'injection de certains vaccins est précédée d'un « test-veine » qui consiste à piquer l'aiguille dans le corps d'un patient, tenir énergiquement la seringue, et tirer sur l'extrémité externe du poussoir de manière à réaliser une aspiration. Si une fine trace de sang apparaît dans la partie de la seringue où est fixée l'aiguille, l'injection ne doit pas être réalisée. L'utilisateur doit piquer à nouveau le patient quelques centimètres à l'écart de la piqûre précédente. Un vaccin ne doit en aucun cas être injecté dans le réseau sanguin.

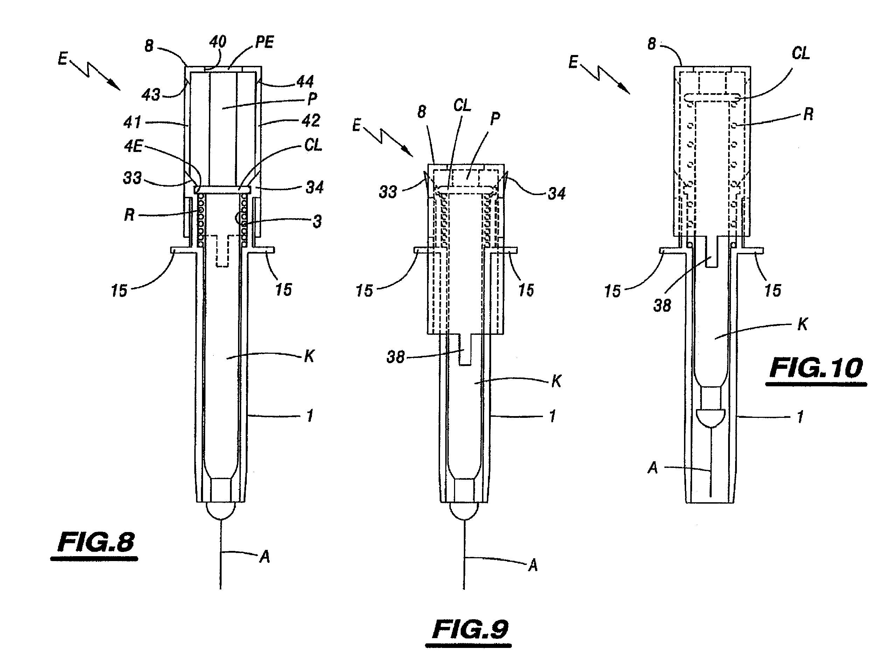

Sur les figures 8 à 13, on a représenté un ensemble de sécurité E selon un troisième mode de réalisation de l'invention. Ce dernier mode de réalisation permet notamment l'utilisation d'une seringue comportant un vaccin.

Sur les figures 8 à 13, les éléments analogues à ceux des figures précédentes, sont désignés par des références identiques.

Sur les figures 8 à 10, on reconnaît les éléments analogues à ceux des modes de réalisation précédents, à savoir le tube 1 comportant le logement 3 dans lequel est disposé le ressort R. L'extrémité proximale du tube 1 est prolongée par deux ergots 33, 34 de préférence diamétralement opposés. En variante, le nombre d'ergots 33, 34 peut être inférieur ou supérieur à deux. Les ergots 33, 34 constituent des moyens de blocage ayant une fonction d'immobilisation du corps K de la seringue par rapport au tube par serrage de la collerette CL de ce corps entre les ergots 33, 34 et le siège d'appui 4E. La fonction des ergots 33, 34 est donc analogue à celles des ergots 6 du premier mode de réalisation de l'invention.

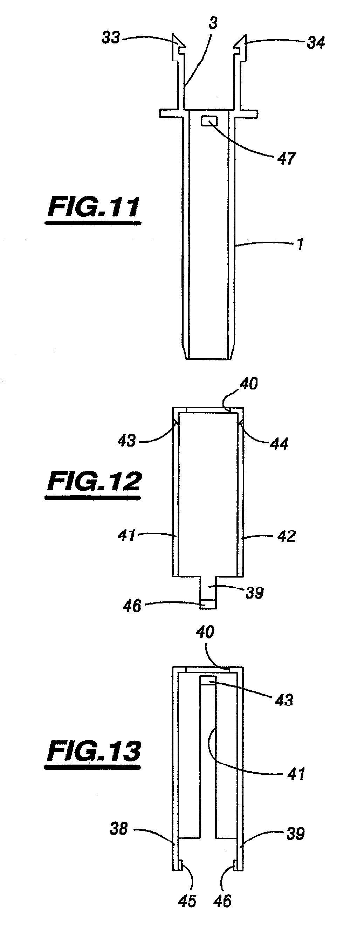

Deux ailettes de préhension 35, 36, analogues aux oreilles 15 du second mode de réalisation, sont venues de moulage avec l'extrémité proximale du tube 1 et disposées à180° l'une de l'autre. Le tube 1 est représenté isolé de l'ensemble E sur la figure 11. Le capuchon 8 (voir notamment figures 12 et 13) comporte dans ce cas deux languettes longitudinales 38, 39, sensiblement diamétralement opposés, prolongeant son extrémité distale. L'extrémité proximale du capuchon 8 est percée d'un trou 40. Le capuchon 8 comporte en outre deux lumières longitudinales verticales 41, 42 décalées de 90° par rapport aux languettes 38, 39.

Les lumières 41, 42 coopèrent par emboîtement avec les ailettes 15 de manière à former des moyens complémentaires d'immobilisation en rotation du capuchon 8 par rapport au tube 1.

Les ergots 33, 34 sont escamotables, de façon à libérer la collerette CL, par coopération avec des rampes 43, 44 limitant les extrémités proximales des lumières 41, 42.

Le trou 40 du capuchon permet le montage du poussoir dans l'ensemble d'injection. Le poussoir P est assujetti au capuchon 8 à l'aide de moyens classiques non représentés.

La figure 8 représente l'ensemble de sécurité E dans une configuration prête à son utilisation, l'aiguille de la seringue faisant saillie à travers l'extrémité distale du tube 1. Partant de cette configuration, l'utilisateur réalise une injection, notamment en intra-musculaire ou en sous-cutanée, en tenant le tube 1 entre l'index et le majeur, grâce aux ailettes 15, tout en appuyant avec le pouce sur l'extrémité externe PE du poussoir. Le capuchon 8 étant assujetti au poussoir P, ce capuchon s'enfonce sous l'effet de la pression du pouce dans le même sens que le poussoir P jusqu'à atteindre la position représentée sur la figure 9.

Lorsque le poussoir P atteint sa position de fin d'injection, telle que représentée sur la figure 9, le capuchon 8 écarte les ergots 33, 34 par coopération des rampes 43, 44 du capuchon 8 avec des ergots 33, 34, ce qui a pour effet de libérer le ressort R.

Le ressort pousse alors le corps de la seringue vide vers l'extrémité proximale du capuchon 8, comme cela est représenté sur la figure 10.

Après libération des ergots 33, 34, la course du capuchon 8 est limitée par des moyens d'encliquetage s'opposant à la force élastique de rappel du ressort R. Ces moyens d'encliquetage comprennent des plots de blocage 45, 46 ménagés sur les extrémités libres des languettes 38, 39 coopérant par emboîtement avec des trous 47, diamétralement opposés, ménagés sur l'extrémité proximale du tube 1 (voir figure 11).

Sur la figure 10, qui illustre la configuration de l'ensemble de sécurité E après utilisation, on voit que l'aiguille est escamotée dans le tube 1.

Dans le troisième mode de réalisation de l'invention, la seringue et l'aiguille sont bien auto-rétractables, le tube 1 constituant une partie stable servant d' appui fixe.

On notera que le tube 1 par ses ailettes 33, 36 assure une stabilité sur une surface non plane.

Les modes de réalisation décrits ci-dessus assurent la rétraction de l'aiguille parfaitement en ligne par rapport à son introduction, tout en remplissant une fonction d'anti-accident pour les personnels soignants.

Toute contagion accidentelle devient impossible, l'extrémité distale du tube 1 ayant un diamètre réduit, mesurant par exemple 9 mm, ce qui interdit l'introduction accidentelle d'un doigt d'un utilisateur. La sécurité de l'ensemble E atteint donc pratiquement 100%, et, conformément au but de l'invention, le déclenchement de la sécurité (escamotage de l'aiguille) est automatique et indépendant de la volonté humaine.

D'autre part, l'escamotage de l'aiguille est réalisé d'une seule main, sans geste spécial, en respectant les règles médicales ainsi que les pratiques des personnels soignants.

L'invention permet aussi bien aux soignants qu'aux personnes devant manipuler, convoyer ou détruire les seringues usagées, d'éviter tout risque d'accidents.

## Revendications

1. Ensemble de sécurité (E) et d'une seringue (S) d'injection de liquide pré-remplie, la seringue comportant un corps tubulaire (K), formant un réservoir pour le liquide, portant une aiguille (A) d'injection du liquide, et un poussoir (P) monté déplaçable dans le corps (K) entre des positions d'attente et de fin d'injection, le corps (K) de la seringue comportant une extrémité proximale munie d'une collerette (CL), l'ensemble comprenant en outre :
- un fourreau tubulaire (1) dans lequel le corps (K) de la seringue (S) est destiné à être logé déplaçable axialement entre une position active, dans laquelle l'aiguille (A) fait saillie à travers une extrémité distale du fourreau (1), et une position de protection, dans laquelle l'aiguille (A) est escamotée dans le fourreau (1),
- des ergots (6) permettant d'immobiliser le corps (K) par rapport au fourreau (1) en position active en s'opposant à la force élastique des moyens de rappel, la collerette étant encliquetée sous les ergots (6),
- des moyens de rappel élastique (R) du corps (K) vers sa position de protection et
- des moyens de blocage (5) destinés à immobiliser le corps (K) par rapport au fourreau (1) en position active en s'opposant à la force élastique des moyens de rappel, ces moyens de blocage étant libérables par des moyens (8) activés lorsque le poussoir (P) est en position de fin d'injection, les moyens de blocage comprenant des moyens escamotables formés par deux pattes (5) diamétralement opposées formées dans la paroi du fourreau (1) et terminées par des ergots (6) escamotables,
- les moyens de libération des moyens de blocage comprennent un capuchon de révolution (8), de préférence en matière plastique, monté coulissant axialement par rapport au fourreau (1) entre deux positions et traversé par le poussoir (P) qui s'étend à travers un orifice (9) ménagé dans l'extrémité proximale de ce capuchon (8),
- deux épaulements (7, 11) complémentaires formant butées, prévus respectivement sur le capuchon (8) et le fourreau (1) pour limiter la course du capuchon (8) après libération des moyens de blocage (5) du corps (K), en s'opposant à la force élastique de rappel des moyens de rappel (R).

2. Ensemble selon la revendication 1, **caractérisé en ce que** les moyens de rappel (R) comprennent un ressort de poussée (R) destiné à venir en appui sur un épaulement interne d'appui (3E) ménagé dans le fourreau (1).

3. Ensemble selon la revendication 2, **caractérisé en ce que** le fourreau (1) comporte un logement interne (3) de révolution dans lequel est disposé le ressort de poussée (R).

4. Ensemble selon la revendication 3, **caractérisé en ce que** ledit logement interne (3) est délimité par l'épaulement interne d'appui (3E) du ressort de poussée (R).

5. Ensemble selon l'une des revendications 1 à 4, **caractérisé en ce qu'**il comporte des moyens (17, 18) pour bloquer la collerette (CL) du corps de seringue (K) après détente des moyens de rappel (R).

6. Ensemble selon l'une des revendications 1 à 5, **caractérisé en ce que** le fourreau (1) et le capuchon (8) comportent des moyens complémentaires d'immobilisation en rotation de l'un par rapport à l'autre.

7. Ensemble selon l'une des revendications 1 à 6, **caractérisé en ce que** le fourreau (1) porte des moyens de préhension externes (1E) destinés à être saisis par les doigts d'un utilisateur pour injecter le liquide par rapprochement axial d'une extrémité de manoeuvre (PE) du poussoir (P) et des moyens de préhension (1E).

8. Ensemble selon l'une des revendications 1 à 7, dans lequel la collerette (CL) est encliquetée après avoir comprimé le ressort (R) dans un premier logement (3) du fourreau (1).
